# EUROPEAN PATENT SPECIFICATION

(11) **EP 0 861 265 B1**
(45) Date of publication and mention of the grant of the patent: **18.09.2002**
(21) Application number: 96935129.5
(22) Date of filing: 31.10.1996
(51) Int. Cl.: C07K 14/47, A23K 1/16, A23L 1/305, A23C 9/12, A61K 38/17

(54) **use of CASEIN FRAGMENTS as GROWTH PROMOTer**
Verwendung von KASEINFRAGMENTEn als WACHSTUMERFÖRDERer
usage des FRAGMENTS DE CASEINE COMME PROMOTEURS DE CROISSANCE

(30) Priority: 31.10.1995 GB 9522302
(43) Date of publication of application: 02.09.1998
(73) Proprietor: Pepsyn Limited, Cambridge CB1 2JE (GB)
(72) Inventor: SMITH, John, Arthur, Liverpool L23 8TN (GB); WILKINSON, Mark, Charles, Irby, Wirral L61 4YW (GB); LIU, Qing-Ming, Liverpool L14 2EL (GB)
(74) Representative: W.P. Thompson & Co.
(86) International application number: GB9602658
(87) International publication number: WO97016460

(56) References cited:
- EP-A- 0 457 565
- DATABASE WPI Section Ch, Week 9435 Derwent Publications Ltd., London, GB; Class B04, AN 94-283276 XP002013699 & JP 06 211 689 A (KANEBO LTD) , 2 August 1994
- DATABASE WPI Section Ch, Week 9201 Derwent Publications Ltd., London, GB; Class B04, AN 92-002669 XP002013698 & JP 03 255 095 A (KANEBO KK) , 13 November 1991
- BIOCHEM.SOC.TRANS., vol. 24, no. 3, 1996, page 342s XP000645809 LIU Q-M E.A.: "A growth factor activity in bovine milk"

## Description

The present invention relates to growth promoters.

It has long been known that milk contains growth promoting activity for cells that is additional to its nutritional content. Thus, Epidermal Growth Factor (EGF) has been identified in human (Shing and Klagsbrun, 1984, Petrides, 1985), rat (Raaberg et al, 1990) swine (Tan et al 1990) and goat (Brown and Blakeley, 1983) milk.

Indeed the EGF present in rat milk has been shown to be significant for the normal development of pups (Oka et al 1983). EGF has not, however, been found in bovine milk (Read 1985). Instead insulin-like growth factor (IGF) I and II (Francis et al, 1986) and bovine colostrum growth factor (BCGF), which is structurally related to Platelet-derived Growth Factor (PDGF) (Shing and Klagsbrun, 1984, Brown and Blakeley, 1984), have been identified.

EP0457565 discloses milk protein hydrolyzates and compositions which are effective in the conditioning of hair and skin.

The applicant has surprisingly discovered that bovine milk contains growth promoting activity for rat mammary fibroblast cell line (Rama 27), which is not significantly stimulated by IGF or PDGF.

Furthermore, they have identified peptide sequences which elicit this growth promoting activity.

The invention relates to a peptide or a salt thereof comprising an amino acid sequence substantially identical to the C-terminal end of the α-S2 casein precursor.

According to a first aspect of the present invention there is provided the use of a peptide or a salt thereof comprising an amino acid sequence of from 9-31 amino acids in length and which is substantially identical to the C-terminal end of an α-S2 casein precursor including homologues thereof in which :
i) one or more of the amino acids Leu, Ile and Val are replaced by one another;
ii) one or more of the amino acids Tyr and Phe are replaced by one another; and/or
iii) one or more of the amino acids Arg and Lys are replaced by one another, for the manufacture of a medicament or foodstuff for promoting growth.

Whilst whole casein protein shows no growth activity, the applicant has identified a number of peptides, derived from the C-terminal end of Bovine α-S2 casein, which elicit growth promoting activity.

Indeed, the applicant has shown this growth promoting activity to be present in at least peptides of 9 to 31 amino acids in length which have been derived from the C-terminal end of Bovine α-S2 casein. It is reasonable to hypothesise that the natural sequence responsible for the growth promoting activity is the sequence comprising the last 9 amino acids of the C-terminal end or an even shorter sequence from within the 9 amino acid sequence, possibly an 8 or 7 amino acid sequence. Indeed, it may be as short as a 3 amino acid sequence.

The bovine α-S2 casein precursor is characterised
in that it has an amino acid sequence:

MKFFIFTCLL AVALAKNTME HVSSSEESII SQETYKQEKN MAINPSKENL CSTFCKEVVR
NANEEEYSIG SSSEESAEVA TEEVKITVDD KHYQKALNEI NQFYQKFPQY LQYLYQGPIV
LNPWDQVKRN AVPITPTLNR EQLSTSEENS KKTVDMESTE VFTKKTKLTE EEKNRLNFLK
KISQRYQKFA LPQYLKTVYQ HQKAMKPWIQ PKTKVIPYVR YL

In three letter codes this translates to:

[CAS2 BOVIN] ALPHA-S2 CASE IN PRECURSOR.
SEQUENCE

MetLysPhePheIlePheThrCysLeuLeu
AlaValAlaLeuAlaLeuAsnThrMetGlu

HisValSerSerSerGluGluSerIleIle
SerGlnGluThrTyrLysGlnGluLysAsn

MetAlaIleAsnProSerLysGluAsnLeu
CysSerThrPheCysLysGluValValArg

AsnAlaAsnGluGluGluTyrSerIleGly
SerSerSerGluGluSerAlaGluValAla

ThrGluGluValLysIleThrValAspAsp
LysHisTyrGlnLysAlaLeuAsnGluIle

AsnGlnPheTyrGlnLysPheProGlnTyr
LeuGlnTyrLeuTyrGlnGlyProIleVal

LeuAsnProTrpAspGlnValLysArgAsn
AlaValProIleThrProThrLeuAsnArg

GluGlnLeuSerThrSerGluGluAsnSer
LysLysThrValAspMetGluSerThrGlu

ValPheThrLysLysThrLysLeuThrGlu
GluGluLysAsnArgLeuAsnPheLeuLys

LysIleSerGlnArgTyrGlnLysPheAla
LeuProGlnTyrLeuLysThrValTyrGln

HisGlnLysAlaMetLysProTrpIleGln
ProLysThrLysValIleProTyrValArg

TyrLeu

The applicant has found that short peptide sequences incorporating the C-terminal sequence -LysValIleProTyrValArgTyrLeu show growth promoting activity.

According to a second aspect of the present invention there is provided a growth factor comprising the amino acid sequence -LysValIleProTyrValArgTyrLeu

Furthermore, comparison of, for example, the last 20 amino acids of the C-terminal sequence for bovine α-S2 casein with those for goat, and sheep shows a high degree of homology as does to a lesser extent the C-terminal amino acid sequence of rabbit and pig α-S2 casein

The sequences for these are set out below.

[CAS2_CAPHI]     ALPHA-S2 CASEIN PRECURSOR (ALPHA-S2-CN).
SEQUENCE
MKFFIFTCLL AVALAKHKME EVSSSEEPIN IFQEIYKQEK NMAIHPRKEK LCTTSCEEVV
RNANEEEYSI RSSSEESAEV APEEIKITVD DKHYQKALNE INQFYQKFPQ YLQYPYQGPI
VLNPWDQVKR NAGPFTPTVN REQLSTSEEN SKKTIDMEST EVFTKKTKLT EEEKNRLNFL
KKISQYYQKF AWPQYLKTVD QHQKAMKPWT QPKTNAIPYV RYL

>pir|S33881|S33881 alphas2-casein E - goat

MKFFIFTCLL AVALAKHKME HVSSSEEPIN IFQEIYKQEK NMAIHPRKEK LCTTSCEEVV
RNANEEEYSI RSSSEESAKV APEEIKITVD DKHYQKALNE INQFYQKFPQ YLQYPYQGPI
VLNPWDQVKR NAGPFTPTVN REQLSTSEEN SKKTIDMEST EVFTKKTKLT EEEKNRLNFL
KKISQYYQKF AWPQYLKTVD QHQKAMKPWT QPKTNAIPYV RYL 223

>gp|S74171|S74171_1 alpha s2-casein C [Capra hircus]

MKFFIFTCLL AVALAKHKME HVSSSEEPIN IFQEIYKQEK NMAIHPRKEK LCTTSCEEVV
RNANEEEYSI RSSSEESAEV APEEIKITVD DKHYQKALNE INQFYQKFPQ YLQYPYQGPI
VLNPWDQVKR NAGPFTPTVN REQLSTSEEN SKKTIDMEST EVFTKKTKLT EEEKNRLNFL
KIISQYYQKF AWPQYLKTVD QHQKAMKPWT QPKTNAIPYV RYL 223

>pir|S39776|S39776 alpha-S2-casein form b precursor - rabbit
>gp|X76909|OCPAS2BCS_1 pre-alpha S2b casein (AA -15 to 167)
[Oryctolagus cuniculus]

MKFFIFTCLL AVALAKPKIE QSSSEETIAV SQEVSPNLEN ICSTACEEPI KNINEVEYVE
VPTEIKDQEF YQKVNLLQYL QALYQYPTVM DPWTRAETKA IPFIRTMQYK QEKDATKHTS
QKTELTEEEK AFLKYLDEMK QYYQKFVFPQ YLKNAHHFQK TMNPWNHVKT IIYQSVPTL 179

[CAS2_SHEEP] ALPHA-S2 CASEIN PRECURSOR.
SEQUENCE
MKFFIFTCLL AVALAKHKME HVSSSEEPIN ISQEIYKQEK NMAIHPRKEK LCTTSCEEVV
RNADEEEYSI RSSSEESAEV APEEVKITVD DKHYQKALNE INQFYQKFPQ YLQYLYQGPI
VLNPWDQVKR NAGPFTPTVN REQLSTSEEN SKKTIDMEST EVFTKKTKLT EEEKNRLNFL
KKISQYYQKF AWPQYLKTVD QHQKAMKPWT QPKTNAIPYV RYL

[CAS2_PIG] ALPHA-S2 CASEIN PRECURSOR.
SEQUENCE
MKFFIFTCLL AVAFAKHEME HVSSSEESIN ISQEKYKQEK NVINHPSKED ICATSCEEAV
RNIKEVGYAS SSSSEESVDI PAENVKVTVE DKHYLKQLEK ISQFYQKFPQ YLQALYQAQI
VMNPWDQTKT SAYPFIPTVI QSGEELSTSE EPVSSSQEEN TKTVDMESME EFTKKTELTE
EEKNRIKFLN KIKQYYQKFT WPQYIKTVHQ KQKAMKPWNH IKTNSYQIIP NLRYF

In three letter code these translate to:

[CAS2 CAPH1] ALPHA-S2 CASEIN PRECURSOR (ALPHA-S2-CN).
SEQUENCE

MetLysPheIlePhePheThrCysLeuLeu
AlaValAlaLeuAlaLysHisLysMetGlu

HisValSerSerSerGlyGlyProIleAsn
IlePheGlnGluIleTyrLysGlnGluLys

AsnMetAlaIleHisProArgLysGluLys
LeuCysThrThrSerCysGluGluValVal

ArgAsnAlaAsnGluGluGluTyrSerIle
ArgSerSerSerGluGluSerAlaGluVal

AlaProGluGluIleLysIleThrValAsp
AspLysHisTyrGlnLysAlaLeuAsnGlu

IleAsnGlnPheTyrGlnLysPheProGln
TyrLeuGlnTyrProTyrGlnGlyProIle

ValLeuAsnProTrpAspGlnValLysArg
AsnAlaGlyProPheThrProThrValAsn

ArgGluGlnLeuSerThrSerGluGluAsn
SerLysLysThrIleAspMetGluSerThr

GluValPheThrLysLysThrLysLeuThr
GluGluGluLysAsnArgLeuAsnPheLeu

LysLysIleSerGlnTyrTyrGlnLysPhe
AlaTrpProGlnTyrLeuLysThrValAsp

GlnHisGlnLysAlaMetLysProTrpThr
GlnProLysThrAsnAlaIleProTyrVal

ArgTyrLeu

>pir/S33881/S33881  alpha S2-casein E goat

MetLysPhePheIlePheThrCysLeuLeu
AlaValAlaLeuAlaLysHisLysMetGlu
HisValSerSerSerGluGluProIleAsn
IlePheGlnGluIleTyrLysGlnGluLys
AsnMetAlaIleHisProArgLysGluLys
LeuCysThrThrSerCysGluGluValVal
ArgAsnAlaAsnGluGluGluTyrSerIle
ArgSerSerSerGluGluSerAlaLysVal
AlaProGluGluIleLysIleThrValAsp
AspLysHisTyrGlnLysAlaLeuAsnGlu
IleAsnGlnPheTyrGlnLysPheProGln
TyrLeuGlnTyrProTyrGlnGlyProIle
ValLeuAsnProTrpAspGlnValLysArg
AsnAlaGlyProPheThrProThrValAsn
ArgGluGlnLeuSerThrSerGluGluAsn
SerLysLysThrIleAspMetGluSerThr
GluValPheThrLysLysThrLysLeuThr
GluGluGluLysAsnArgLeuAsnPheLeu
LysLysIleSerGlnTyrTyrGlnLysPhe
AlaTrpProGlnTyrLeuLysThrValAsp
GlnHisGlnLysAlaMetLysProTrpThr
GlnProLysThrAsnAlaIleProTyrVal

ArgTyrLeu  223

>pir/S74171/S74171 1  alpha S2-casein C [Capra hircus]

MetLysPhePheIlePheThrCysLeuLeu
AlaValAlaLeuAlaLysHisLysMetGlu
HisValSerSerSerGluGluProLIeAsn
IlePheGlnGluIleTyrLysGlnGluLys
AsnMetAlaIleHisProArgLysGluLys
LeuCysThrThrSerCysGluGluValVal
ArgAsnAlaAsnGluGluGluTyrSerIle
ArgSerSerSerGluGluSerAlaGluVal
AlaProGluGluIleLysIleThrValAsp
AspLysHisTyrGlnLysAlaLeuAsnGlu
IleAsnGlnPheTyrGlnLysPheProGln
TyrLeuGlnTyrProTyrGlnGlyProIle
ValLeuAsnProTrpAspGlnValLysArg
AsnAlaGlyProPheThrProThrValAsn
ArgGluGlnLeuSerThrSerGluGluAsn
SerLysLysThrIleAspMetGluSerThr
GluValPheThrLysLysThrLysLeuThr
GluGluGluLysAsnArgLeuAsnPheLeu
LysIleIleSerGlnTyrTyrGlnLysPhe
AlaTrpProGlnTyrLeuLysThrValAsp
GlnHisGlnLysAlaMetLysProTrpThr
GlnProLysThrAsnAlaIleProTyrVal
ArgTyrLeu  223

>pir/S39776/S39776  alpha-S2- Casein form b precursor -
rabbit
>gp/X76909/OCPAS2BCS 1 pre-alpha S"b casein (AA
-15 to 167)
[Oryctolagus cuniculus]

MetLysPhePheIlePheThrCysLeuLeu
AlaValAlaLeuAlaLysProLysIleGlu
GlnSerSerSerGluGluThrIleAlaVal
SerGlnGluValSerProAsnLeuGluAsn
IleCysSerThrAlaCysGluGluProIle
LysAsnIleAsnGluValGluTyrValGlu
ValProThrGluIleLysAspGlnGluPhe
TyrGlnLysValAsnLeuLeuGlnTyrLeu
GlnAlaLeuTyrGlnTyrProThrValMet
AspProTrpThrArgAlaGluThrLysAla
IleProPheIleArgThrMetGlnTyrLys
GlnGluLysAspAlaThrLysHisThrSer
GlnLysThrGluLeuThrGluGluGluLys
AlaPheLeuLysTyrLeuAspGluMetLys
GlnTyrTyrGlnLysPheValPheProGln
TyrLeuLysAsnAlaHisHisPheGlnLys
ThrMetAsnProTrpAsnHisValLysThr
IleIleTyrGlnSerValProThrLeu
179

[CAS2 SHEEP]  ALPHA -S2 CASEIN PRECURSOR
SEQUENCE.

MetLysPhePheIlePheThrCysLeuLeu
AlaValAlaLeuAlaLysHisLysMetGlu
HisValSerSerSerGluGluProIleAsn
IleSerGlnGluLIeTyrLysGlnGluLys
AsnMetAlaIleHisProArgLysGluLys
LeuCysThrThrSerCysGluGluValVal
ArgAsnAlaAspGluGluGluTyrSerIle
ArgSerSerSerGluGluSerAlaGluVal
AlaProGluGluValLysLIeThrValAsp
AspLysHisTyrGlnLysAlaLeuAsnGlu
IleAsnGlnPheTyrGlnLysPheProGln
TyrLeuGlnTyrLeuTyrGlnGlyProIle
ValLeuAsnProTrpAspGlnValLysArg
AsnAlaGlyProPheThrProThrValAsn
ArgGluGlnLeuSerThrSerGluGluAsn
SerLysLysThrIleAspMetGluSerThr
GluValPheThrLysLysThrLysLeuThr
GluGluGluLysAsnArgLeuAsnPheLeu
LysLysIleSerGlnTyrTyrGlnLysPhe
AlaTrpProGlnTyrLeuLysThrValAsp
GlnHisGlnLysAlaMetLysProTrpThr
GlnProLysThrAsnAlaIleProTyrVal
ArgTyrLeu

[CAS2 PIG]   ALPHA-S2 CASEIN PRECURSOR.
SEQUENCE

MetLysPhePheIlePheThrCysLeuLeu
AlaValAlaPheAlaLysHisGluMetGlu
HisValSerSerSErGluGluSerIleAsp
IleSerGlnGluLysTyrLysGlnGluLys
AsnValIleAsnHisProSerLysGluAsp
IleCysAlaThrSerCysGluGluAlaVal
ArgAsnIleLysGluValGluTyrAlaSer
SerSerSerSerGluGluSerValAspIle
ProAlaGluAsnValLysValThrValGlu
AspLysHisTyrLeuLysGlnLeuGluLys
IleSerGlnPheTyrGlnLysPheProGln
TyrLeuGlnAlaLeuTyrGlnAlaGlnIle
ValMetAsnProTrpAspGlnThrLysThr
SerAlaTyrProPheIleProThrValIle
GlnSerGlyGluGluLeuSerThrSerGlu
GluProValSerSerSerGlnGluGluAsn
ThrLysThrValAspMetGluSerMetGlu
GluPheThrLysLysThrGluLeuThrGlu
GluGluLysAsnArgLIeLysPheLeuAsn
LysLIeLysGlnTyrTyrGlnLysPheThr
TrpProGlnTyrIleLysThrValHisGln
LysGlnLysAlaMetLysProTrpAsnHis
IleLysThrAsnSerTyrGlnIleIlePro
AsnLeuArgTyrPhe

It will be apparent from this that the C-terminal sequence can vary from species to species and that consequently whilst the preferred sequences comprise those derived from the C-terminal end of the bovine α-S2 casein those of the other species might be used.

Furthermore, due to the similar nature of some amino acids it is possible that minor substitutions may have little effect on the functioning of the sequence.

Thus, for example, Leucine, isoleucine and valine may be interchanged. Tyrosine and phenylalanine may be interchanged, and arginine and lysine may be interchanged

The significance of the discovery is that a peptide supplement which can promote growth can be added to food or drink products, for both human or animal consumption.

According to a further aspect of the present invention there is provided a food or drink product comprising a peptide or salt thereof of the invention.

Preferably the food or drink product is an infant formula or an animal feed. It may be in liquid or powder form.

Whilst it is possible to synthetically produce peptides according to the present invention it would be desirable to produce the peptide in situ from cows milk.

According to a further aspect of the present invention milk is treated with an enzyme to break the casein in the milk into smaller fragments containing the active peptide or a salt thereof of the invention.

Preferably the enzyme is a protease and more particularly one which cleaves lysine cross-bonds. More preferably still it is plasmin or trypsin.

The invention will be further described by way of example only with reference to the following examples:

### EXAMPLE 1

The growth promoting activity of different milk types was determined by precipitating caseins and assaying the supernatants for their ability to stimulate the incorporation of [3H] thymidine into the DNA of Rama 27 cells by known methodology (Smith et al, 1984).

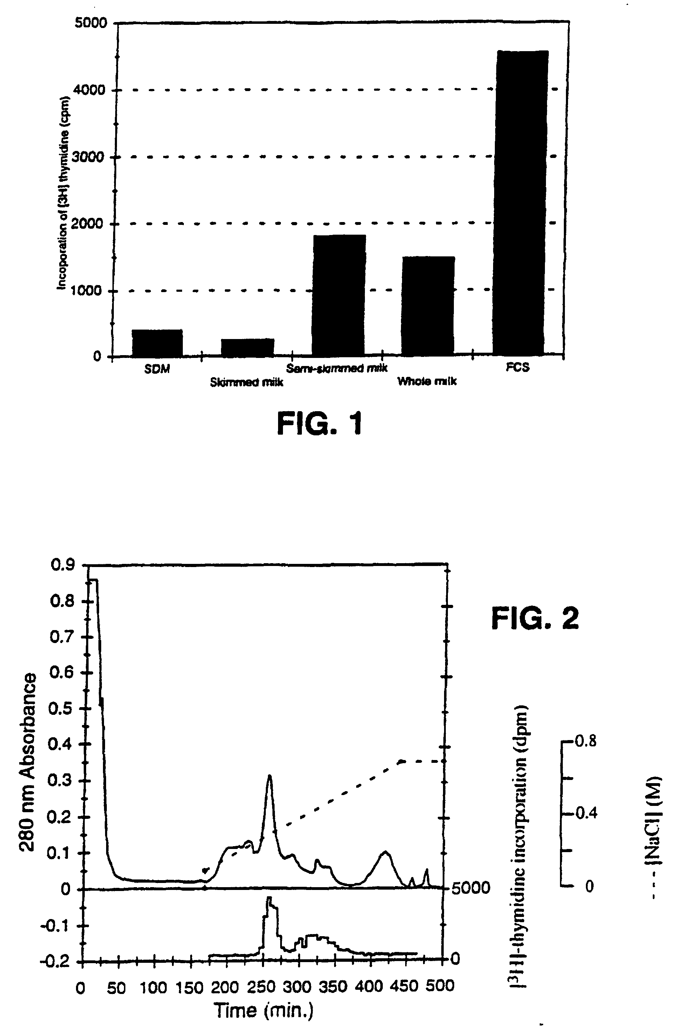

The results of the tests are illustrated in Fig 1. which shows the growth-promoting activity of different milk types. Three sorts of commercial milks were acidified to precipitate the caseins and assayed for their growth promoting activity. The greatest activity was found in semi-skimmed milk. SDM (step down medium) represents the negative control and FCS (foetal calf serum) represents the positive control.

### EXAMPLE 2

5 litres of semi-skimmed milk was made to pH 3.0 with HCl and left for 2 hours at 4°C. It was centrifuged in a Sorvall RC5B centrifuge at 9000 rpm in a GS3 rotor for 40 min, and the supernatant (approximately 3.6 litres) was poured through glass wool to remove fat. Solid (NH₄)₂SO₄ was added slowly to the supernatant with stirring at 4°C to a concentration of 22% (w/v), and was left for 2 hours at 4°C without stirring. Precipitated protein was removed by centrifugation as above. To the supernatant was added further (NH₄)₂SO₄ to a concentration of 35% (w/v) and the precipitate recovered as above. The precipitate was redissolved in 1600ml distilled water and dialyzed against running tap water overnight, then against 20mM NaH₂PO₄,pH6.0, for 8 hours.

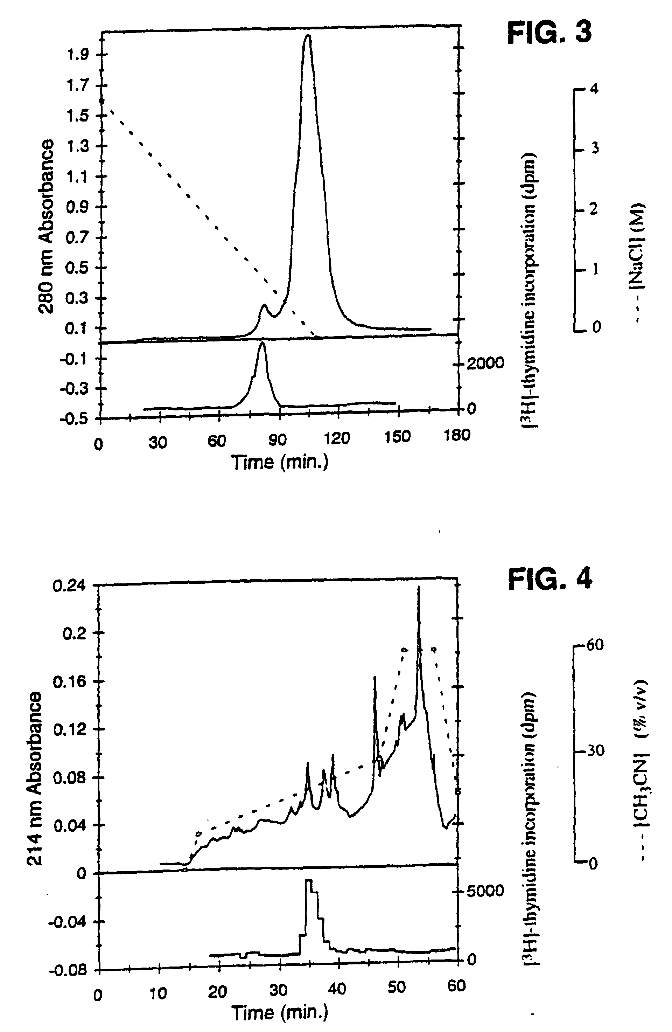

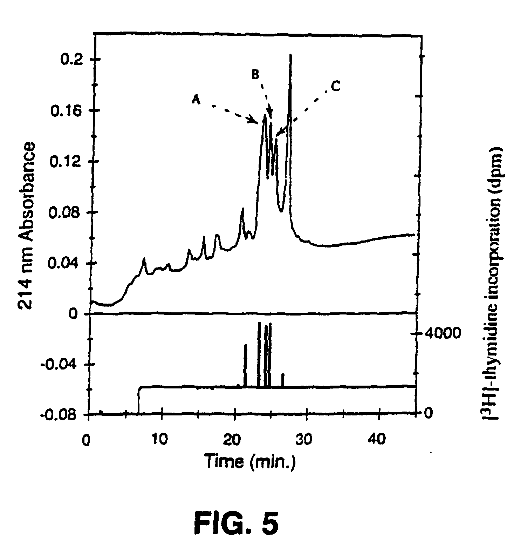

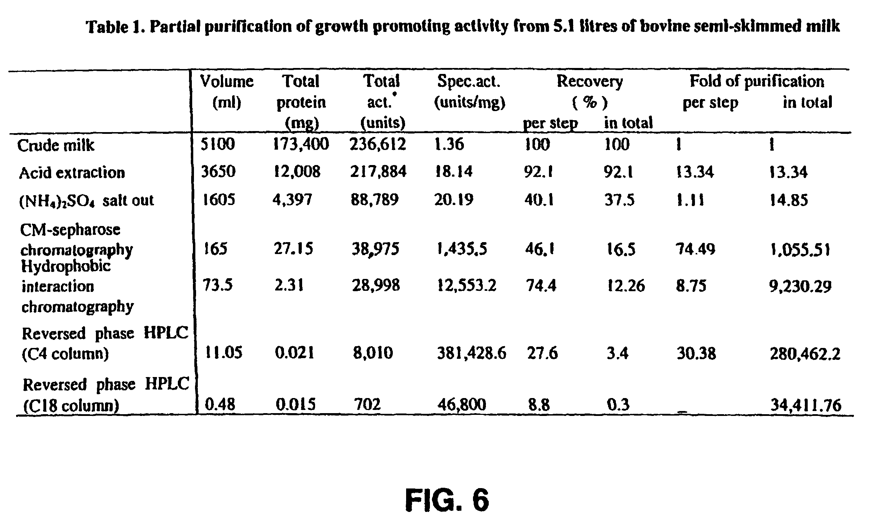

The active fractions were obtained using a series of chromatographic techniques as outlined in (i) to (iv) below:
(i) The active fraction prepared as above was subjected to CM-Sepharose chromatography. It was added to a column of CM-Sepharose (10cm x 5cm id, Pharmacia) that had been pre-equilibrated with 20mM Sodium phosphate buffer pH6.0. After loading, the column was washed with 500ml of 50mM NaCl in the same buffer. Protein was eluted with a 1500ml linear gradient of 0.1 to 0.7M NaCl in 20mM sodium phosphate buffer pH 6.0. The bioactive fractions eluted at 0.28M NaCl and approximately 0.4M NaCl - see Fig. 2. In Fig 2 the upper panel shows the absorbance of the protein at 280nm and the lower panel shows the activity (The incorporation of ³H- thymidine into DNA). The sample was from material precipitating between 22 to 35% (NH₄)₂SO₄. After being redissolved and dialyzed it was loaded into the column (10 cm x 5 cm) with 0.05 M NaCl in 20mM NaH₂PO₄, pH 6.0. The eluting gradient was 0.1-0.7 M NaCl in 20 mM NaH₂PO₄,pH6. The flowrate was 5ml/min, the fraction size was 25 ml each. Two activities eluted at 0.28 M NaCl and 0.34-0.45 M NaCl respectively. The high absorbance at 280 nm at the beginning of the trace indicates the amount of unbound protein. The fraction-eluted at 0.28 M NaCl was used for further purification.
(ii) The active fractions from the above separation were subjected to hydrophobic interaction chromatography. It was made 3.7M with NaCl in 20mM NaH₂PO₄, pH6.5, and applied to a butyl Sepharose column (8.6 cm x 2.5 cm id) that had been preequilibrated with 4M NaCl in 20mM NaH₂PO₄, pH6.5. Protein was eluted with a decreasing gradient of NaCl as indicated in Fig 3. In Fig. 3 the upper panel shows the absorbance of the protein at 280 nm and the lower panel shows the activity (The incorporation of ³H-thymidine into DNA). The sample was from the early activity after CM-Sepharose chromatography. The column (2.5 cm x 8.6 cm, butyl bonded Sepharose) had been equilibrated with 4 M NaCl in 20 mM NaH₂PO₄, pH 6.5. The flowrate was 3.5 ml/min and fraction size was 3.5 ml. The activity eluted at 1.6 M NaCl, just before the major protein peak.
(iii)The active fractions from the hydrophobic interaction column were subjected to Reversed Phased HPLC-1 chromatography. It was applied in 8 batches to a butyl reversed phase column (Brownlee, 300A pore size, 7µm particle size, 25cm x 4.6mm id) that had been pre-equilibrated with 0.1% TFA. After washing the column with 0.1% TFA, protein was eluted with a gradient of acetonitrile (far uv grade, Rathburns, Walkerburn, Scotland) as indicated in Fig 4. In Fig. 4 the upper panel shows the absorbance of the protein at 214 nm and the lower panel shows the activity (The incorporation of ³H-thymidine into DNA). The sample was from the activity after hydrophobic interaction chromatography. The column (250 cm x 4.6 mm, C4) had been equilibrated with 0.1% TFA. The flow rate was 0.7 ml/min and fraction size was 0.7 ml. The eluting gradient was 10 to 30% acetonitrile in 0.1% TFA in 30 min. The activity eluted at 23% acetonitrile.
(iv) The active fractions were then subjected to reversed phase HPLC-2 chromatography. The mitogenic fractions from all 8 batches of the above reversed phase chromatograms were pooled and concentrated on a centrifugal drier to a total volume of 100µl. This concentrated material was loaded onto a C18 reversed phase column (ODS ultrasphere, Beckman) which had been pre-equilibrated with 0.1% TFA, and was eluted with a shallow gradient of 20 to 40% acetonitrile, 0.1% TFA over 45 min, at a flow rate of 0.2ml/min. Absorbance was monitored at 214nm, and material from each peak of absorbance was collected separately by hand - see Fig 5. In Fig. 5 the upper panel shows the absorbance of the protein at 214 nm and the lower panel shows the activity (The incorporation of ³H-thymidine into DNA). The sample was from the activity after reversed phase HPLC-1. The column (ODS) had been equilibrated with 0.1% TFA. The flowrate was 0.2 ml/min. Each absorption peak at 214 nm was collected manually. The eluting gradient was 20 to 40% acetonitrile in 0.1% TFA in 45 min. The peaks A,B,C (arrows) were all active.

The purified proteins (peaks A,B,C) obtained in step (iv) were then analysed.

Protein content was measured by the binding of Coomassie Blue according to the Bio-Rad protocol, using bovine gamma globulin as standard. Peptide quantification of fractions separated by HPLC was by their absorbance at 214nm, using cytochrome c and lysozyme as standards.

The protein fractions A,B,C, of the casein digest where assayed for their ability to stimulate the incorporation of [3H] thymidine into the DNA of Rama 27 cells exactly as described previously.

The results are illustrated in Table 1 which shows the growth promoting activity of progressively purified fractions of α-S2 casein.

The peptides from the peaks B and C of reversed phase HPLC-2 were then sequenced. They were found to be a nested series of sequences of 5 peptides. They corresponded to the C-terminus of bovine α-S2 casein. The peak C was solely ThrLysValIleProTyrValArgTyrLeu, the other sequences were from peak B.

The sequences of the peaks are identified below:

Sequence 1 LysValIIeProTyrValArgTyrLeu (peak B)

Sequence 2 ThrLysValIIeProTyrValArgTyrLeu (peak C)

Sequence 3 LysThrLysValIIeProTyrValArgTyrLeu (peak B)

Sequence 4

AlaMetLysProTrpIleGlnProLysThrLysValIleProTyrValArgTyrLeu
(peak B)
Sequence 5
ProGlnTyrLeuLysThrValTyrGlnHisGlnLysAlaMetLysProTrpIleGlnPro
LysThrLysValIleProTyrValArgTyrLeu (peak B)

To ascertain that the activity was not due to impurities identical peptide sequences were synthesized on a Milligen/Biosearch 9050 peptide synthesizer (Millipore, Watford) using Fmoc chemistry and pentafluorophenyl esters according to the standard protocol.

Of these initially only LysValIleProTyrValArgTyrLeu showed bioactivity, but after storage in PBS all the peptides acquired a low level of mitogenicity. The activity of LysValIleProTyrValArgTyrLeu was substantially increased when maintained at alkaline pH. By way of contrast alpha-casein was inactive in the mitogenic assay. On digestion with trypsin, activity in the assay was generated, which was separable by reversed phase HPLC from that due to trypsin itself.

The example described herein demonstrates that the growth factor activity of milk is largely due to C-terminal fragments of α-S2 casein.

Given the activity of the peptide it is expected that the addition of from 0.1µg to 10µg, more particularly about 1 µg of peptide to 250g of feed or drink will provide good growth promotion activity.

However, in order to maintain the activity the synthetic peptides should be stored in alkaline conditions, preferably at about pH 13.

### SEQUENCE LISTING

SEQUENCE I.D. No 1
   LENGTH: 9 amino acids
   TYPE: Amino acid
   SEQUENCE: LysValIleProTyrValArgTyrLeu
SEQUENCE I.D. No 2
   LENGTH: 10 amino acids
   TYPE: Amino acids
   SEQUENCE: ThrLysValIleProTyrValArgTyrLeu
SEQUENCE I.D. No 3
   LENGTH: 11 amino acids
   TYPE: Amino acids
   SEQUENCE: LysThrLysValIleProTyrValArgTyrLeu
SEQUENCE I.D. No 4
   LENGTH: 19 amino acids
   TYPE: Amino acids
   SEQUENCE: AlaMetLysProTrpIleGlnProLysThrLysValIleProTyrValArgTyrLeu
SEQUENCE I.D. No 5
   LENGTH: 31 amino acids
   TYPE: Amino acids
   SEQUENCE:

ProGlnTyrLeuLysThrValTyrGlnHisGlnLysAlaMetLysProTrpIleGlnPro

LysThrLysValIleProTyrValArgTyrLeu

## Claims

1. Use of a peptide or a salt thereof comprising an amino acid sequence of from 9-31 amino acids in length and which is substantially identical to the C-terminal end of an α-S2 casein precursor including homologues thereof in which
i) one or more of the amino acids Leu, Ile and Val are replaced by one another;
ii) one or more of the amino acids Tyr and Phe are replaced by one another; and/or
iii) one or more of the amino acids Arg and Lys are replaced by one another, for the manufacture of a medicament or foodstuff for promoting growth.

2. Use of a peptide or salt thereof as claimed in claim 1, wherein the peptide is derived from bovine, goat, sheep, rabbit or pig α-S2 casein or is a synthesised equivalent or a homologue thereof in which
i) one or more of the amino acids Leu, Ile and Val are replaced by one another;
ii) one or more of the amino acids Tyr and Phe are replaced by one another; and/or
iii) one or more of the amino acids Arg and Lys are replaced by one another.

3. Use of a peptide or a salt thereof as claimed in claim 2, wherein the peptide is derived from bovine α-S2 casein or is a synthesised equivalent or a homologue thereof in which
i) one or more of the amino acids Leu, lie and Val are replaced by one another;
ii) one or more of the amino acids Tyr and Phe are replaced by one another; and/or
iii) one or more of the amino acids Arg and Lys are replaced by one another.

4. Use of a peptide or a salt thereof as claimed in any of the preceding claims, in which the peptide comprises 9 amino acids.

5. Use of a peptide or a salt thereof as claimed in any of the preceding claims comprising the amino acid sequence: Lys ValIleProTry ValArgTyrLeu or a homologue thereof in which
i) one or more of the amino acids Leu, Ile and Val are replaced by one another;
ii) one or more of the amino acids Tyr and Phe are replaced by one another; and/or
iii) one or more of the amino acids Arg and Lys are replaced by one another.

6. Use of a peptide as claimed in any of claims 1 to 5, in which the peptide has the sequence:

LysValIleProTyrValArgTyrLeu.

7. Use of a peptide as claimed in any of claims 1 to 5, in which the peptide has the sequence:

ThrLysValIleProTyrValArgTyrLeu.

8. Use of a peptide as claimed in any of claims 1 to 5, in which the peptide has the sequence:

LysThrLysValIleProTyrValArgTyrLeu.

9. Use of a peptide as claimed in any of claims 1 to 5, in which the peptide has the sequence:

AlaMetLysProTrpIleGlnProLysThrLys ValIleProTyr ValArgTyrLeu.

10. Use of a peptide as claimed in any of claims 1 to 5, in which the peptide has the sequence:

ProGlnTyrLeuLysThrValTyrGlnHisGlnLysAlaMetLysProTrpIleGlnProLysThr
LysValIleProTyrValArgTyrLeu.

11. Use of a peptide as claimed in any of the preceding claims in which the foodstuff is an infant formula or an animal feed.

12. Use of a peptide as claimed in any of the preceding claims in which the medicament or foodstuff is a liquid or powder.

13. Use of a peptide as claimed in any of the preceding claims, in which the medicament or foodstuff comprises whole milk or semi-skimmed milk.

14. Use of a peptide as claimed in any of the preceding claims, in which the medicament or foodstuff has an alkaline pH.

15. Use of a peptide as claimed in any of the preceding claims, in which the peptide is present in an effective amount.

16. Use of a peptide as claimed in claim 15, wherein the effective amount is 0.1 to 10µg to 250g of medicament or foodstuff.

17. A food or drink product comprising a peptide or a salt thereof comprising an amino acid sequence of from 9-31 amino acids in length and which is substantially identical to the C-terminal end of an α-S2 casein precursor or a homologue thereof in which
i) one or more of the amino acids Leu, lie and Val are replaced by one another;
ii) one or more of the amino acids Tyr and Phe are replaced by one another; and/or
iii) one or more of the amino acids Arg and Lys are replaced by one another.

18. A method of producing a medicament or foodstuff comprising a growth promoting peptide comprises treating milk with an enzyme to break milk casein present in the milk into one or more peptides comprising an amino acid sequence of from 9-31 amino acids in length and which is substantially identical to the C-terminal end of the α-S2 casein precursor.

## Patentansprüche

1. Verwendung eines Peptids oder eines Salzes davon, das eine Aminosäuresequenz von 9 - 31 Aminosäuren in Länge umfasst und die im Wesentlichen identisch mit dem C-terminalen Ende eines α-S2-Casein-Precursors, einschließlich Homologa davon ist, worin
i) eine oder mehrere der Aminosäuren Leu, Ile und Val miteinander ausgetauscht werden;
ii) eine oder mehrere der Aminosäuren Tyr und Phe miteinander ausgetauscht werden und/oder
iii) eine oder mehrere der Aminosäuren Arg und Lys miteinander ausgetauscht werden, für die Herstellung eines Medikamentes oder Nahrungsmittels zur Wachstumsförderung.

2. Verwendung eines Peptids oder Salzes davon nach Anspruch 1, worin sich das Peptid von α-S2-Casein vom Rind, von der Ziege, vom Schaf, vom Kaninchen oder vom Schwein herleitet oder ein synthetisiertes Äquivalent oder ein Homologon davon ist, worin
i) eine oder mehrere der Aminosäuren Leu, Ile und Val miteinander ausgetauscht werden;
ii) eine oder mehrere der Aminosäuren Tyr und Phe miteinander ausgetauscht werden und/oder
iii) eine oder mehrere der Aminosäuren Arg und Lys miteinander ausgetauscht werden.

3. Verwendung eines Peptids oder eines Salzes davon nach Anspruch 2, worin sich das Peptid von α-S2-Casein vom Rind herleitet oder ein synthetisiertes Äquivalent oder ein Homologon davon ist, worin
i) eine oder mehrere der Aminosäuren Leu, Ile und Val miteinander ausgetauscht werden;
ii) eine oder mehrere der Aminosäuren Tyr und Phe miteinander ausgetauscht werden und/oder
iii) eine oder mehrere der Aminosäuren Arg und Lys miteinander ausgetauscht werden.

4. Verwendung eines Peptids oder eines Salzes davon nach einem der vorangehenden Ansprüche, worin das Peptid 9 Aminosäuren umfasst.

5. Verwendung eines Peptids oder eines Salzes davon nach einem der vorangehenden Ansprüche, das die Aminosäuresequenz Lys Val Ile Pro Try Val Arg Tyr Leu oder ein Homologon davon umfasst, worin
i) eine oder mehrere der Aminosäuren Leu, Ile und Val miteinander ausgetauscht werden;
ii) eine oder mehrere der Aminosäuren Tyr und Phe miteinander ausgetauscht werden und/oder
iii) eine oder mehrere der Aminosäuren Arg und Lys miteinander ausgetauscht werden.

6. Verwendung eines Peptids nach einem der Ansprüche 1 bis 5, worin das Peptid die folgende Sequenz aufweist:

Lys Val Ile Pro Tyr Val Arg Tyr Leu.

7. Verwendung eines Peptids nach einem der Ansprüche 1 bis 5, worin das Peptid die folgende Sequenz aufweist:

Thr Lys Val Ile Pro Tyr Val Arg Tyr Leu.

8. Verwendung eines Peptids nach einem der Ansprüche 1 bis 5, worin das Peptid die folgende Sequenz aufweist:

Lys Thr Lys Val Ile Pro Tyr Val Arg Tyr Leu.

9. Verwendung eines Peptids nach einem der Ansprüche 1 bis 5, worin das Peptid die folgende Sequenz aufweist:

Ala Met Lys Pro Trp Ile Gln Pro Lys Thr Lys Val Ile Pro Tyr Val Arg Tyr Leu.

10. Verwendung eines Peptids nach einem der Ansprüche 1 bis 5, worin das Peptid die folgende Sequenz aufweist:

Pro Gln Tyr Leu Lys Thr Val Tyr Gln His Gln Lys Ala Met Lys Pro Trp Ile Gln Pro Lys
Thr Lys Val Ile Pro Tyr Val Arg Tyr Leu.

11. Verwendung eines Peptids nach einem der vorangehenden Ansprüche, worin das Nahrungsmittel eine Kinderformel oder ein Tierfutter ist.

12. Verwendung eines Peptids nach einem der vorangehenden Ansprüche, worin das Medikament oder Nahrungsmittel eine Flüssigkeit oder ein Pulver ist.

13. Verwendung eines Peptids nach einem der vorangehenden Ansprüche, worin das Medikament oder das Nahrungsmittel Vollmilch oder teilentrahmte Milch umfasst.

14. Verwendung eines Peptids nach einem der vorangehenden Ansprüche, worin das Medikament oder Nahrungsmittel einen alkalischen pH aufweist.

15. Verwendung eines Peptids nach einem der vorangehenden Ansprüche, worin das Peptid in einer wirksamen Menge vorliegt.

16. Verwendung eines Peptids nach Anspruch 15, worin die wirksame Menge 0,1 bis 10 µg bis 250 µg des Medikamentes oder Nahrungsmittels beträgt.

17. Nahrungsmittel- oder Getränkeprodukt, das ein Peptid oder ein Salz davon umfasst, das eine Aminosäuresequenz von 9 - 31 Aminosäuren in Länge umfasst und die im Wesentlichen mit dem C-terminalen Ende eines α-S2-Casein-Precursors oder eines Homologons davon identisch ist, worin
i) eine oder mehrere der Aminosäuren Leu, Ile und Val miteinander ausgetauscht werden,
ii) eine oder mehrere der Aminosäuren Tyr und Phe miteinander ausgetauscht werden, und/oder
iii) eine oder mehrere der Aminosäuren Arg und Lys miteinander ausgetauscht werden.

18. Verfahren zum Herstellen eines Medikamentes oder Nahrungsmittels, das ein wachstumsförderndes Peptid umfasst, das das Behandeln der Milch mit einem Enzym umfasst, um das in der Milch vorliegende Milchcasein in ein Peptid oder mehrere Peptide aufzuspalten, das eine Aminosäuresequenz von 9 - 31 Aminosäuren in Länge umfasst und die im Wesentlichen identisch mit dem C-terminalen Ende des α-S2-Casein-Precursors ist.

## Revendications

1. Utilisation d'un peptide ou d'un sel de celui-ci comprenant une séquence d'acides aminés d'une longueur allant de 9 à 31 acides aminés et qui est sensiblement identique à l'extrémité C-terminale d'un précurseur de caséine α-S2, y compris les homologues de celui-ci, dans lequel
i) un ou plusieurs parmi les acides aminés Leu, Ile et Val sont remplacés l'un par l'autre ;
ii) un ou plusieurs parmi les acides aminés Tyr et Phe sont remplacés l'un par l'autre ; et/ou
iii) un ou plusieurs parmi les acides aminés Arg et Lys sont remplacés l'un par l'autre, pour la fabrication d'un médicament ou d'un produit alimentaire pour favoriser la croissance.

2. Utilisation d'un peptide ou d'un sel de celui-ci selon la revendication 1, dans laquelle le peptide est dérivé d'une caséine α-S2 de bovin, de chèvre, de mouton, de lapin ou de porc, ou est un équivalent de synthèse ou un homologue de celle-ci, dans lequel
i) un ou plusieurs parmi les acides aminés Leu, Ile et Val sont remplacés l'un par l'autre ;
ii) un ou plusieurs parmi les acides aminés Tyr et Phe sont remplacés l'un par l'autre ; et/ou
iii) un ou plusieurs parmi les acides aminés Arg et Lys sont remplacés l'un par l'autre.

3. Utilisation d'un peptide ou d'un sel de celui-ci selon la revendication 2, dans laquelle le peptide est dérivé d'une caséine α-S2 de bovin, ou est un équivalent de synthèse ou un homologue de celle-ci, dans lequel
i) un ou plusieurs parmi les acides aminés Leu, Ile et Val sont remplacés l'un par l'autre ;
ii) un ou plusieurs parmi les acides aminés Tyr et Phe sont remplacés l'un par l'autre ; et/ou
iii) un ou plusieurs parmi les acides aminés Arg et Lys sont remplacés l'un par l'autre.

4. Utilisation d'un peptide ou d'un sel de celui-ci selon l'une quelconque des revendications précédentes, dans laquelle le peptide comprend 9 acides aminés.

5. Utilisation d'un peptide ou d'un sel de celui-ci selon l'une quelconque des revendications précédentes, comprenant la séquence d'acides aminés : LysValIleProTryValArgTyrLeu. ou un homologue de celui-ci, dans lequel
i) un ou plusieurs parmi les acides aminés Leu, Ile et Val sont remplacés l'un par l'autre ;
ii) un ou plusieurs parmi les acides aminés Tyr et Phe sont remplacés l'un par l'autre ; et/ou
iii) un ou plusieurs parmi les acides aminés Arg et Lys sont remplacés l'un par l'autre.

6. Utilisation d'un peptide selon l'une quelconque des revendications 1 à 5, dans laquelle le peptide possède la séquence :

LysValIleProTyrValArgTyrLeu.

7. Utilisation d'un peptide selon l'une quelconque des revendications 1 à 5, dans laquelle le peptide possède la séquence :

ThrLysValIleProTyrValArgTyrLeu.

8. Utilisation d'un peptide selon l'une quelconque des revendications 1 à 5, dans laquelle le peptide possède la séquence :

LysThrLysValIleProTyrValArgTyrLeu.

9. Utilisation d'un peptide selon l'une quelconque des revendications 1 à 5, dans laquelle le peptide possède la séquence :

AlaMetLysProTrpIleGlnProLysThrLysValIleProTyrValArgTyrLeu.

10. Utilisation d'un peptide selon l'une quelconque des revendications 1 à 5, dans laquelle le peptide possède la séquence :

ProGlnTyrLeuLysThrValTyrGlnHisGlnLysAlaMetLysProTrpIleGlnProLysThrLysVal
IleProTyrValArgTyrLeu.

11. Utilisation d'un peptide selon l'une quelconque des revendications précédentes, dans laquelle le produit alimentaire est une formule de lait pour nourrisson ou un aliment pour animaux.

12. Utilisation d'un peptide selon l'une quelconque des revendications précédentes, dans laquelle le médicament ou le produit alimentaire est un liquide ou une poudre.

13. Utilisation d'un peptide selon l'une quelconque des revendications précédentes, dans laquelle le médicament ou le produit alimentaire comprend du lait entier ou du lait demi-écrémé.

14. Utilisation d'un peptide selon l'une quelconque des revendications précédentes, dans laquelle le médicament ou le produit alimentaire est de pH alcalin.

15. Utilisation d'un peptide selon l'une quelconque des revendications précédentes, dans laquelle le peptide est présent en une quantité efficace.

16. Utilisation d'un peptide selon la revendication 15, dans laquelle la quantité efficace va de 0,1 à 10 µg pour 250 g de médicament ou de produit alimentaire.

17. Produit alimentaire ou de boisson comprenant un peptide ou un sel de celui-ci comprenant une séquence d'acides aminés d'une longueur allant de 9 à 31 acides aminés et qui est sensiblement identique à l'extrémité C-terminale d'un précurseur de caséine α-S2, ou d'un homologue de celui-ci, dans lequel
i) un ou plusieurs parmi les acides aminés Leu, Ile et Val sont remplacés l'un par l'autre ;
ii) un ou plusieurs parmi les acides aminés Tyr et Phe sont remplacés l'un par l'autre ; et/ou
iii) un ou plusieurs parmi les acides aminés Arg et Lys sont remplacés l'un par l'autre.

18. Méthode de production d'un médicament ou d'un produit alimentaire comprenant un peptide favorisant la croissance, comprenant le traitement de lait par une enzyme pour cliver la caséine du lait présente dans le lait en un ou plusieurs peptides comprenant une séquence d'acides aminés d'une longueur allant de 9 à 31 acides aminés et qui est sensiblement identique à l'extrémité C-terminale d'un précurseur de caséine α-S2.
